# EUROPÄISCHE PATENTSCHRIFT

(11) **EP 1 682 020 B1**
(45) Veröffentlichungstag und Bekanntmachung des Hinweises auf die Patenterteilung: **17.10.2007**
(21) Anmeldenummer: 03818482.6
(22) Anmeldetag: 08.09.2003
(51) Int. Cl.: A61B 17/74, A61B 17/80

(54) **VORRICHTUNG ZUR KNOCHENFIXATION**
BONE FIXING DEVICE
DISPOSITIF DE FIXATION OSSEUSE

(43) Veröffentlichungstag der Anmeldung: 26.07.2006
(73) Patentinhaber: Synthes GmbH, 4436 Oberdorf (CH)
(72) Erfinder: DUTOIT, Christof, CH-4500 Solothurn (CH); FRENK, André, CH-4805 Brittnau (CH); CHELIUS, Philippe, F-10390 Clerey (FR)
(74) Vertreter: Lusuardi, Werther
(86) Internationale Anmeldenummer: PCT/CH2003/000604
(87) Internationale Veröffentlichungsnummer: WO 2005/023127

(56) Entgegenhaltungen:
- EP-B- 0 515 828
- US-A- 5 336 224
- US-B1- 6 348 052
- PATENT ABSTRACTS OF JAPAN vol. 2000, no. 02, 29. Februar 2000 (2000-02-29) & JP 11 299804 A (HOMUZU GIKEN:KK), 2. November 1999 (1999-11-02)

## Beschreibung

Die Erfindung bezieht sich auf eine Trochanterstabiliserungsvorrichtung gemäss dem Oberbegriff des Anspruchs 1 sowie auf eine Hüftschraubenvorrichtung gemäss dem Oberbegriff des Anspruchs 20.

Solche Vorrichtungen werden beispielsweise zur Versorgung von Frakturen am proximalen Femur, insbesondere von instabilen trochantären Frakturen vom Typ gemäss AO-Klassifikation 31-A2 und 31-A3 eingesetzt.

Eine solche Vorrichtung ist aus der EP-B 0 515 828 bekannt, welche eine Hülsenlasche und eine damit lösbar verbindbare Trochanterstabilisierungsplatte umfasst. Nachteilig bei dieser bekannten Vorrichtung ist, dass
- die Trochanterstabilisierungsplatte relativ starr ist und sich kaum an die jeweilige Anatomie anpassen lässt;
- keine winkelstabilen Schrauben eingesetzt werden können;
- die Verwendung von Kortikalisschrauben ebenfalls kaum möglich ist, da die Kortex im Bereich des grossen Trochanters sehr dünn ist und kaum eine Verankerung der Kortikalisschrauben erlaubt;
- die Fixation mit Cerclage unter Umständen ungenügend ist. Der grosse Trochanter kann bei dieser bekannten Platten nur mit Hilfe eines Cerclage-Drahtes fixiert werden. Diese Fixationsart ist aber unter Umständen ungenügend um eine Dislokation des grossen Trochanters nach kranial zu verhindern, da der am grossen Trochanter angreifende Muskel gluteus medius mit einer Kraft zieht, welche etwa einmal dem Körpergewicht entspricht; und
- die Modularität eingeschränkt ist. Mit der bekannten Trochanterstabilisierungsplatte kann den unterschiedlichen Anatomien und Frakturkonfigurationen nicht immer Rechnung getragen werden.

JP-A-11-299804 beschreibt eine Vorrichtung mit dem Merkmalen des Oberbegriffs von Anspruch 1.

Aus dem deutschen Gebrauchsmuster U1 87 06 912.1 ist eine Kleinknochenplatte zur Versorgung von Frakturen des Schädel- und Gesichtsskelettes bekannt, d.h. für eine andere Anwendung als zur Fixierung von Knochenfragmenten im gelenksnahen Bereich. Entsprechend ihrer Anwendung ist diese bekannte Knochenplatte geradlinig, L-förmig oder als Doppel-T ausgebildet und weist eigentlich keine zentrale Platte auf, sondern ist insgesamt als konventionelle Knochenplatte ausgebildet. Eine Anwendung zur Fixierung von Knochenfragmenten im gelenksnahen Bereich ist somit mit dieser bekannten Platte nicht gegeben.

Hier will die Erfindung Abhilfe schaffen. Der Erfindung liegt die Aufgabe zugrunde, eine Trochanterstabilisierungsvorrichtung zu schaffen, welche an die Oberfläche des grossen Trochanters anpassbare Knochenstabilisierungsmittel umfasst.

Die Erfindung löst die gestellte Aufgabe mit einer Trochanterstabilisierungsvorrichtung, welche die Merkmale des Anspruchs 1 aufweist, sowie einer Hüftschraubenvorrichtung, welche die Merkmale des Anspruchs 20 aufweist.

Die durch die Erfindung erreichten Vorteile sind im wesentlichen darin zu sehen, dass dank der erfindungsgemässen Vorrichtung:
- eine Medialisierung des Femurschaftes verhindert werden kann, da die als Trochanterstabilisierungsplatte ausgebildeten Knochenstabilisierungsmittel als laterale Abstützung dienen;
- die Arme der Trochanterstabilisierungsplatte erlauben es, die Fragmente des grossen Trochanters zusammenzuhalten und zu fixieren;
- die Gewindelöcher im proximalen Teil der Trochanterstabilisierungsplatte erlauben eine stabile Fixierung von Fragmenten des grossen Trochanters mit winkelstabilen Schrauben, d.h. Knochenschrauben deren Schraubenkopf beispielsweise mittels eines Gewindes in der Trochanterstabilisierungsplatte verschraubt ist und sich daher gegenüber der Platte nicht verdrehen oder verschieben lässt. Eine Dislokation des abgetrennten grossen Trochanters nach kranial ist dadurch vermeidbar. Ansonsten kann die Biomechanik des proximalen Femurs stark beeinträchtigt sein;
- die Trochanterstabilisierungsplatte modular ausgestaltet ist, indem sie einfach an die jeweilige Anatomie angeformt und zurechtgeschnitten werden kann. Dabei erfolgt das Zurechtschneiden der Platte ohne Bildung von Graten, da diese nur um die Plattenlöcher herum getrennt werden kann; und
- der Dynamisierungsmechanismus der Hüftschraube nicht beeinträchtigt wird. Eine zusätzliche Schraube zur Verhinderung einer Rotation des Femurkopfes kann weiterhin eingesetzt werden. Im Gegensatz zur bekannten Trochanterplatte kann bei der vorliegenden Trochanterplatte die Antirotationsschraube an einer beliebigen Stelle angeordnet sein.

In einer bevorzugten Ausführungsform weist die zentrale Platte der Knochenstabilisierungsmittel eine Länge L und eine Breite B < L sowie eine Längsachse auf, während die Arme knochenplattenförmig ausgebildet sind und eine Breite b < B aufweisen. Die Arme umfassen mindestens eine ein Loch aufweisende Hülse, wobei zwischen je zwei Hülsen oder zwischen einer Hülse und der zentralen Platte Stege angeordnet sind. Der wesentliche Vorteil dieser Ausgestaltung der Arme liegt darin, dass durch die Wahl geeigneter Abmessungen sich eine Länge der Stege ergibt, welche es ermöglicht, dass zum Trennen der Stege ein Standardschneideinstrumentes, beispielsweise ein Schneidinstrument (AO Nr. 329.142) aus dem Set der Kalkaneusplatte verwendbar ist.

Vorzugsweise weisen dazu die Hülsen einen Aussendurchmesser D_{A} zwischen 6 bis 10 mm auf, während der Abstand A zwischen zwei Löchern zwischen 10 mm und 15 mm beträgt.

In einer anderen Ausführungsform beträgt der Winkel zwischen zwei benachbarten Armen mindestens 30°, vorzugsweise mindestens 40°. Damit ist der Vorteil erreichbar, dass einerseits ebenfalls das oben erwähnte Schneidwerkzeug verwendet werden kann, und dass andererseits die Schrauben in möglichst regelmässigen Abständen in den grossen Trochanter eingebracht werden können.

Vorzugsweise schliessen dazu die Arme mit der Längsachse der zentralen Platte einen Winkel α ein, welcher in einem Bereich von ±5° bis ±115°, vorzugsweise von ±10° bis ±110° liegt.

Erfindungsgemäß ist mindestens ein Teil der Löcher in den Armen mit einem Innengewinde versehen. Durch diese Ausgestaltung sind die folgenden Vorteile erreichbar:
- winkelstabile Verbindung der mit einem zum Innengewinde komplementären Aussengewinde am Kopf versehenen Knochenfixationsmittel, insbesondere Knochenschrauben in den Armen der Knochenstabilisierungsmittel;
- im Bereich des grossen Trochanters ist die Kortikalis sehr dünn. Daher lassen sich ausser bei sehr jungen Patienten hier kaum normale Kortikalisschrauben ohne Gewindekopf einbringen. Mit winkelstabilen Schrauben hingegen kann auch in sehr weichem Knochen ohne harte Kortikalis die Relativbewegung zwischen den fixierten Fragmenten verhindert werden;
- Schrauben ohne Gewindekopf werden meistens bikortikal verwendet, das heisst, sie müssen bis in die gegenüberliegende Kortikalis greifen. Winkelstabile Schrauben hingegen können auch monokortikal eingesetzt werden, wie es im Bereich des grossen Trochanters notwendig ist;
- die winkelstabilen Schrauben erlauben es, die Platte vom Knochen auf Distanz zu halten. Dadurch wird die Durchblutung des Periostes weniger beeinträchtigt, was zu einer schnelleren Knochenheilung führen kann.

Vorzugsweise ist das Innengewinde in den Löchern konisch ausgebildet, denn Kopfverriegelungsschrauben mit einem zylindrischen Gewinde sind wesentlich schwieriger im richtigen Winkel in der Platte einzuschrauben.

In einer weiteren Ausführungsform ist die senkrecht zur zentralen Platte gemessene Dicke "D" der Arme im Bereich der Löcher gegenüber der Dicke "d" der die einzelnen Löcher verbindenden Stege grösser. Damit sind die Vorteile erreichbar, dass
- Bei einer Verformung, insbesondere einer Biegung der Arme bei einer Anpassung an die anatomischen Verhältnisse werden durch diese Ausgestaltung die Innengewinde in den Hülsen nicht deformiert, so dass deren Funktion durch die Verformung der Arme nicht beeinträchtigt wird;
- Die Arme sind exakt beim Übergang von den Stegen zu den Hülsen trennbar, so dass durch das Durchtrennen keine Grate auf der Plattenoberfläche entstehen;
- Durch die geringere Wandstärke lassen sich die Arme besser trennen.

In wiederum einer weiteren Ausführungsform sind die Arme quer zur Längsachse der zentralen Platte durch Stege verbunden, wodurch der Vorteil erreichbar ist, dass die Hülsen so miteinander verbunden werden können, dass die Knochenfragmente zusammengehalten werden. Ein Verschieben der Fragmente des grossen Trochanters nach kranial soll vermieden werden. Der Muskel gluteus medius zieht mit einer Kraft den grossen Trochanter nach kranial, welche rund einmal dem Körpergewicht entspricht.

Die Knochenstabilisierungsmittel können so ausgestaltetes sein, dass die Länge der Arme mindestens 6 mm, vorzugsweise mindestens 8 mm beträgt. Jeder Arm weist mindestens ein Loch auf. Der Abstand zwischen zwei Löchern des gleichen Arms kann weniger als 6 mm, vorzugsweise zwischen 3,5 mm und 4,5 mm betragen. Fernen kann der Abstand zwischen dem - von der zentralen Platte aus gesehen - periphersten Loch und der Längsachse zwischen 10 mm und 40 mm betragen.

In einer anderen Ausführungsform bestehen die Knochenstabilisierungsmittel aus rostfreiem Stahl mit einer Bruchdehnung von mindestens 40%. Vorzugsweise beträgt die Zusammensetzung der Metallegierung 17.0 - 19.0 Prozent Chrom, 13.0 bis 15 Prozent Nickel und 2.7 und 3.0 Prozent Molybdän. Die Knochenstabilisierungsmittel sind vorzugsweise aus einem weichen Material hergestellt, so dass die Arme beim Anbiegen an die Knochenoberfläche nicht brechen.

In wiederum einer anderen Ausführungsform weist die zentrale Platte an ihren Längsseiten senkrecht zur Platte stehende Führungsschienen auf. Vorzugsweise entspricht die Breite der Knochenplatte dem Abstand der Führungsschienen, so dass die Knochenstabilisierungsmittel zusammen mit der zentralen Platte beispielsweise auf einer Knochenplatte parallel zu dieser verschiebbar sind.

In einer weiteren Ausführungsform weisen die Knochenstabilisierungsmittel und die Knochenplatte aufeinander abgestimmte Befestigungsperforationen auf, in welche Knochenfixationsmittel, insbesondere Knochenschrauben einführbar sind, so dass die Knochenstabilisierungsmittel und die Knochenplatte gemeinsam mit dem Knochen verbindbar sind.

In einer bevorzugten Ausführungsform der erfindungsgemässen Hüftschraubenvorrichtung umfasst diese eine Ausführungsform der oben aufgeführten Trochanterstabilisierungsvorrichtung und ein Befestigungselement zur Einführung in die Hülse, welche als Hüftschraube oder als Spiralklinge ausgebildet ist.

Ein Ausführungsbeispiel der Erfindung, welches zugleich das Funktionsprinzip erläutert, ist in der Zeichnung dargestellt und wird im folgenden näher beschrieben.

Es zeigen
Fig. 1 eine Aufsicht auf die Knochenstabilisierungsmittel einer Ausführungsform der erfindungsgemässen Trochanterstablilierungsvorrichtung;
Fig. 2 eine Seitenansicht der Knochenstabilisierungsmittel gemäss Fig. 1;
Fig. 3 eine Vorderansicht der Knochenstabilisierungsmittel gemäss Fig. 1;
Fig. 4 ein Querschnitt durch einen Abschnitt eines Armes der Knochenstabilisierungsmittel gemäss Fig. 1 bis 3;
Fig. 5 eine Aufsicht auf einen der Arme der Knochenstabilisierungsmittel gemäss Fig. 1 bis 3; und
Fig. 6 einen Längsschnitt durch eine Trochanterstabiliserungsvorrichtung mit Knochenstabilisierungsmitteln gemäss Fig. 1 bis 5 und einer eine Knochenplatte umfassenden Hülsenlasche, welche am Femur montiert ist.

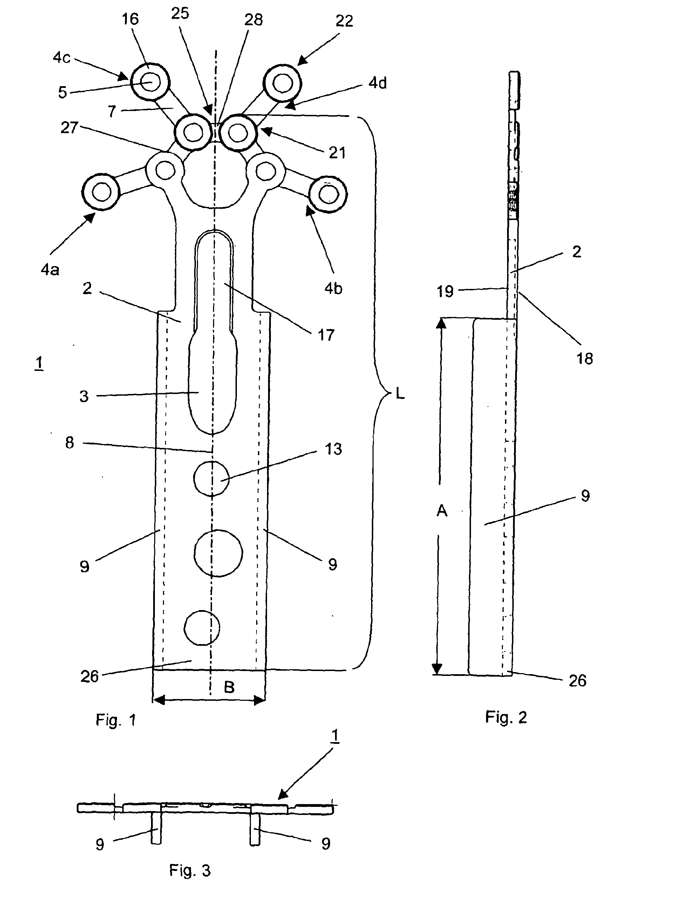

Gemäss den Fig. 1 bis 4 umfassen die Knochenstabilisierungsmittel 1 im wesentlichen eine zentrale Platte 2 mit einer Längsachse 8, einer äusseren, dem Knochen 10 abgewandte Oberfläche 18, einer inneren Oberfläche 19 und vier periphere, gegenüber der Längsachse 8 abgewinkelte, oder abgekröpfte Arme 4. Die zentrale Platte 2 weist parallel zur Längsachse 8 eine Länge L auf und quer dazu eine Breite B, wobei B < L ist. Ferner ist die zentrale Platte 2 mit einem die Platte 2 von der äusseren Oberfläche 18 bis zur inneren Oberfläche 19 durchdringenden Langloch 3, mehreren ebenfalls durchgehenden Befestigungsperforationen 13 und einer ebenfalls durchgehenden, in das Langloch 3 mündenden Öffnung 17 versehen. Die Arme 4 sind am ersten, die Längsachse 8 schneidenden Ende 25 der Platte 2 angeordnet. Dabei ist die Platte 2 an ihrem ersten Ende 25 gabelartig ausgestaltet und an den Gabelspitzen 27 mit je einer ein Loch 5 aufweisenden Hülse 16 versehen und umfasst endständig einen bogenartigen Quersteg 28, weicher die beiden Gabelspitzen 27 verbindet und zwei je ein Loch 5 aufweisende Hülsen 16 aufweist. Die festen Enden 21 zweier Arme 4a;4b sind mit den Hülsen 16 an den Gabelspitzen 27 verbunden während die festen Enden 21 der zwei anderen Arme 4c;4d mit den Hülsen 16 am Quersteg 28 verbunden sind. Dabei sind die Arme 4a bis 4d so angeordnet, dass die zwei mit den Hülsen 16 der Gabelspitzen 27 verbundenen Arme 4a;4b mit der Längsachse 8 einen Winkel von ca. 110° einschliessen während die zwei mit den Hülsen 16 am Quersteg 28 verbundenen Arme 4c;4d mit der Längsachse 8 einen Winkel von ca. 35° einschliessen.

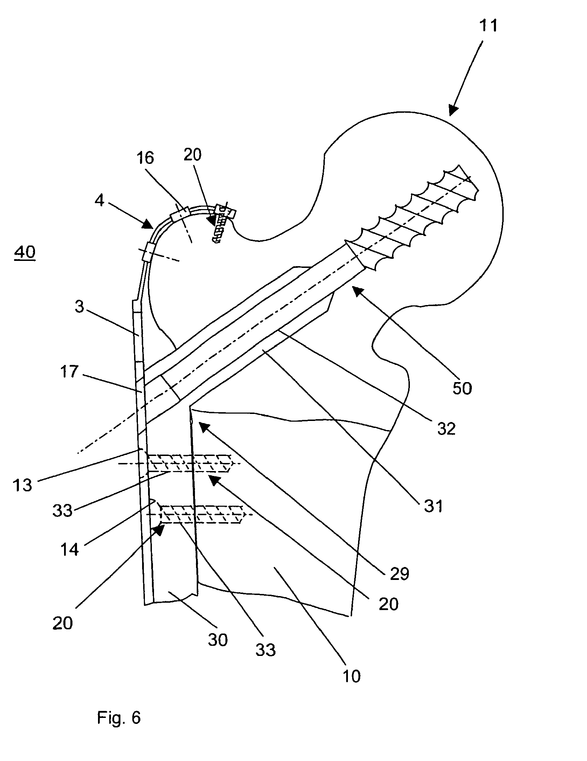

Die Befestigungsperforationen 13 dienen zur Aufnahme von Knochenfixationsmitteln 20, insbesondere von Knochenschrauben mittels welcher die Knochenstabilsierungsmittel 1 zusammen mit der Knochenplatte 30 einer Hülsenlasche 29 (Fig. 6) am Knochen 10 befestigbar ist. Die Öffnung 17 ist als Langloch ausgebildet, so dass bei bedarf eine zusätzliche Knochenschraube (nicht gezeichnet) eingebracht werden kann. Durch dieses zusätzliche Knochenschraube lässt sich ein Verdrehen des Hüftgelenkkopfes relativ zum Femur verhindern. Das Befestigungselement 50 (Fig. 6), welches beispielsweise als Hüftschraube oder Spiralklinge ausgebildet sein kann, kann bei axialen Verschiebungen mit seinem hinteren Ende in das Langloch 3 ausweichen.

Die Arme 4 setzen sich hier aus je einer am freien Ende 22 angeordneten Hülse 16 und je einem zwischen der Hülse 16 und dem festen Ende 21 angeordneten Steg 7 zusammen, wobei die Hülsen 16 eine Dicke D aufweisen und die Stege 7 eine geringere Dicke d haben, so dass die Stege 7 biegbar sind, ohne dass sich dabei die Hülsen 16 und insbesondere die Innengewinde 6 darin verformen. Die Löcher 5 in den Hülsen 16 sind konisch ausgestaltet und mit einem konischen Innengewinde 6 versehen.

Ferner sind an der zentralen Platte 2 parallel zur Längsachse 8 auf einer Länge A vom zweiten Ende 26 der Platte 2 gemessen, zwei Führungsschienen 9 angebracht, mittels welcher die zentrale Platte 2 parallel zu ihrer Längsachse 8 auf der am Knochen 10 befestigten Knochenplatte 30 der Hülsenlasche 29 (Fig. 6) verschiebbar ist bis die Arme 4 an der Oberfläche des grossen Trochanters 12 (Fig. 6) anliegen.

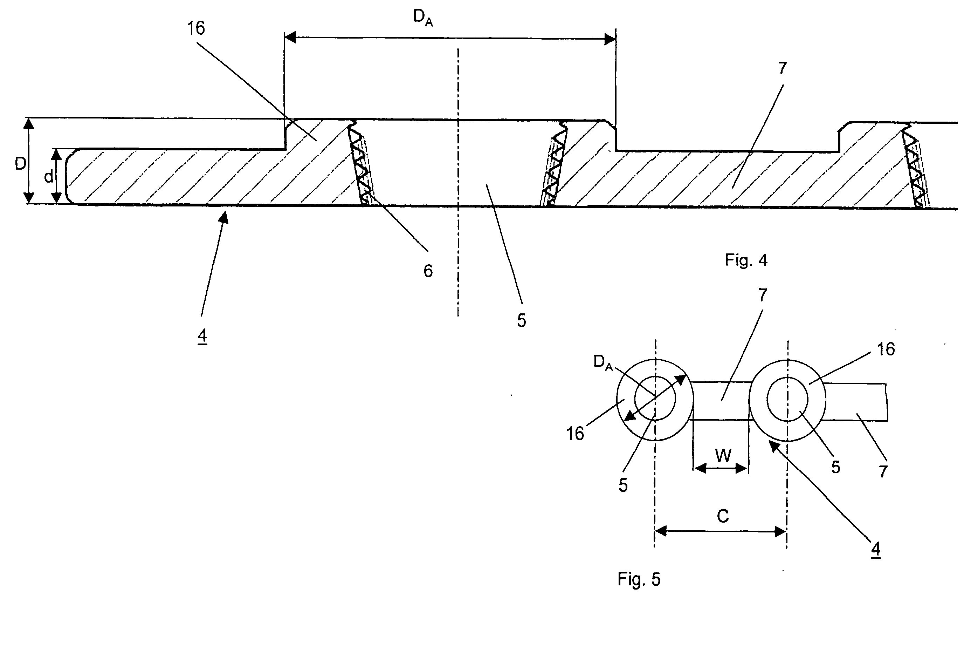

Ein Ausschnitt aus einem der Arme 4 ist in Fig. 5 dargestellt. Damit ein im Handel erhältliches Schneidinstrument, beispielsweise das Schneidinstrument (AO Nr. 329.142) aus dem Set der Kalkaneusplatte verwendet werden kann, sind die Abmessungen der Hülsen 16 mit den Löchern 5 und den Stegen 7 wie folgt gewählt:
- der Abstand C zwischen zwei Zentren zweier benachbarter Löcher 5 beträgt 14 mm;
- der Abstand W zwischen zwei benachbarten Hülsen 16 beträgt 6 mm; und
- der Aussendurchmesser D_{A} der Hülsen 16 beträgt 8 mm.

Die in Fig. 6 dargestellte Ausführungsform der Trochanterstabilisierungsvorrichtung 40 besteht im wesentlichen aus einer konventionellen Hülsenlasche 29, wie sie zur Versorgung von Schenkelhals- und insbesondere trochantären Frakturen des Femur verwendet werden, sowie der als Trochanterstabilierungsplatte ausgebildeten, zentralen Platte 2 mit vier peripheren, gegenüber der Längsachse 8 der zentralen Platte 2 abgewinkelten Armen 4 (Fig. 1 - 4). Die Stege 7 (Fig. 1) der Arme 4 können derart gebogen werden, dass die Arme 4 an der Oberfläche des grossen Trochanters 12 anliegen. Die Hülsenlasche 29 umfasst eine mit dem Knochen 10, insbesondere dem Femurschaft verbindbare, parallel zur Längsachse des Femurschaftes verlaufende, mit einer Anzahl Befestigungsperforationen 33 versehene Knochenplatte 30 und eine daran in einem Winkel stehende Führungshülse 31 mit einer Zentralbohrung 32, durch welche ein Befestigungselement 50, insbesondere eine Hüftschraube oder Spiralklinge durchführbar ist. Die Befestigungsperforationen 33 sind vorzugsweise versetzt angeordnet und mit einer Ansenkung 14 versehen. Zur Fixation der Knochenplatte 30 am Knochen sind ebenfalls als Knochenschrauben ausgestaltete Knochenfixationsmittel 20 einsetzbar. Zur besseren anatomischen Adaption an die gewölbte Knochenoberfläche ist die Knochenplatte 30 als an die Knochenoberfläche adaptierter Hohlzylindersektor ausgestaltet.

Mittels der Führungsschienen 9 können die Knochenplatte 30 und die zentrale Platte 2 solange relativ zueinander und parallel zur Längsachse 8 verschoben werden, bis die Befestigungsperforationen 13 in der zentralen Platte 2 mit den Befestigungsperforationen 33 an der Knochenplatte 30 fluchten, so dass die zentrale Platte 2 mittels einem Teil der in der Knochenplatte 30 einzuschraubenden Knochenfixationsmittel 20, insbesondere Knochenschrauben an der Hülsenlasche 29 befestigbar ist.

Die konventionelle Operationstechnik zur Implantation der Knochenplatte 30 besteht darin, dass
- mittels eines Instrumentes in einem Arbeitsvorgang in latero-medialer Richtung unterhalb des grossen Trochanters mehrere Bohrungen unterschiedlichen Durchmessers zum Einbringen des Befestigungselementes 50 und der an der Hülsenlasche 29 angebrachten Führungshülse 31 in das Zentrum des Schenkelhalses angebracht werden;
- anschliessend das Befestigungselement 50 in den Schenkelhals eingebracht wird, wobei mittels eines Zielgerätes die korrekte Einschraubtiefe bestimmt wird;
- danach die Führungshülse 31 der Hülsenlasche 29 über das Befestigungselement 50 geschoben wird;
- die Hülsenlasche 29 mit Hilfe von als Knochenschrauben ausgebildeten Knochenfixationsmitteln 20 am Knochenschaft fixiert wird, wobei die erste und die dritte Befestigungsperforation 33 der Hülsenlasche 29 leer gelassen werden;
- die Arme 4 der Trochanterplatte mit Hilfe eines geeigneten Instrumentariums entsprechend der vorliegenden Frakturkonfiguration zurechtgeschnitten und zurechtgebogen werden;
- anschliessend die zentrale Platte 2 über die durch die zentrale Platte 2 und die Knochenplatte 30 durchgehenden Befestigungsperforationen 13;33 mit Hilfe von als Knochenschrauben ausgebildeten Knochenfixationsmitteln 20 an die bereits implantierte Hülsenlasche 29 montiert wird;
- bei Bedarf über die Öffnung 17 in den Knochenstabilisierungsmitteln 1 eine Knochenschraube mit einem Durchmesser von mindestens 6,5 mm eingebracht werden kann, welche eine Rotationsbewegung zwischen dem Kopffragment und dem Femurschaft verhindern soll. Die Knochenstabilisierungsmittel 1 kann dabei als Gegenhalt dienen, um das Kopffragment nach lateral zu ziehen und so den Frakturspalt zu schliessen;
- die Knochenfragmente des grossen Trochanters anschliessend mit Hilfe von Knochenfixationsmitteln 20, insbesondere mit winkelstabilen Knochenschrauben mit Kopfgewinden fixiert werden können. Durch die winkelstabile Verankerung der Knochenschrauben in den Löchern 5 der Knochenstabilisierungsmitteln 1 wird eine Relativbewegung zwischen den Schrauben und somit zwischen den Knochenfragmenten verhindert.

Die Trochanterstabilisierungsplatte kann dank ihrer konstruktiven Steifigkeit aus einem ziemlich dünnen Blech gefertigt werden.

## Patentansprüche

1. Trochanterstabilisierungsvorrichtung (40), insbesondere zur Fixation von Knochenfragmenten im Bereich des Hüftgelenkes (11) oder zur Fixierung des grossen Trochanters (12), mit
A) Knochenstabilisierungsmitteln (1), welche eine zentrale Platte (2) mit mindestens einer Befestigungsperforation (13) zur Aufnahme eines Knochenfixationsmittels (20) umfassen; und
B) einer longitudinalen Knochenplatte (30), an welcher eine in einem Winkel stehende Hülse (31) zur Aufnahme eines in den Bereich des Hüftgelenkes (11) einzuführenden Befestigungselementes (50) befestigt ist, wobei
C) der zentralen Platte (2) mindestens drei periphere Arme (4) entspringen; und
D) jeder periphere Arm (4) über mindestens ein Loch (5) zur Aufnahme eines Knochenfixationsmittels (20) verfügt; **dadurch gekennzeichnet, dass**
E) mindestens ein Teil der Löcher (5) mit einem Innengewinde (6) versehen ist.

2. Trochanterstabilisierungsvorrichtung (40) nach Anspruch 1, **dadurch gekennzeichnet, dass** die zentrale Platte (2) zwei an ihren Längsseiten angeordnete, senkrecht zur zentralen Platte (2) stehende Führungsschienen (9) umfasst.

3. Trochanterstabilisierungsvorrichtung (40) nach Anspruch 2, **dadurch gekennzeichnet, dass** die Breite der Knochenplatte (30) dem Abstand der beiden Führungsschienen (9) entspricht, derart, dass die zentrale Platte (2) auf die Knochenplatte (30) aufschiebbar und in Längsrichtung darauf verschieblich ist.

4. Trochanterstabilisierungsvorrichtung (40) nach einem der Ansprüche 1 bis 3, **dadurch gekennzeichnet, dass** die zentrale Platte (2) eine Länge L und eine Breite B < L sowie eine Längsachse (8) aufweist.

5. Trochanterstabilisierungsvorrichtung (40) nach Anspruch 4, **dadurch gekennzeichnet, dass** die Arme (4) knochenplattenförmig ausgebildet sind und eine Breite b < B . aufweisen.

6. Trochanterstabilisierungsvorrichtung (40) nach einem der Ansprüche 1 bis 5, **dadurch gekennzeichnet, dass** die Arme (4) mindestens zwei die Löcher (5) aufweisende Hülsen (16) umfassen und die Hülsen (16) mittels Stegen (7) miteinander verbunden sind.

7. Trochanterstabilisierungsvorrichtung (40) nach Anspruch 6, **dadurch gekennzeichnet, dass** die Hülsen (16) einen Aussendurchmesser D_{A} zwischen 6 bis 10 mm aufweisen.

8. Trochanterstabilisierungsvorrichtung (40) nach einem der Ansprüche 1 bis 7, **dadurch gekennzeichnet, dass** der Abstand C zwischen zwei Löchern (5) zwischen 10 mm und 15 mm liegt.

9. Trochanterstabilisierungsvorrichtung (40) nach einem der Ansprüche 1 bis 8, **dadurch gekennzeichnet, dass** der Winkel zwischen den zwei benachbarten Armen (4) mindestens 30°, vorzugsweise mindestens 40° beträgt.

10. Trochanterstabilisierungsvorrichtung (40) nach einem der Ansprüche 1 bis 9, **dadurch gekennzeichnet, dass** die Arme (4) mit der Längsachse (8) einen Winkel α einschliessen, welcher in einem Bereich von 5° bis 90° liegt.

11. Trochanterstabilisierungsvorrichtung (40) nach Anspruch 10, **dadurch gekennezeichnet, dass** der Winkel α in einem Bereich von 10° bis 80° liegt.

12. Trochanterstabilisierungsvorrichtung (40) nach Anspruch 11, **dadurch gekennzeichnet, dass** das Innengewinde (6) konisch ist.

13. Trochanterstabilisierungsvorrichtung (40) nach einem der Ansprüche 1 bis 12, **dadurch gekennzeichnet, dass** die senkrecht zur Platte (2) gemessene Dicke "D" der Arme (4) im Bereich der Löcher (5) gegenüber der Dicke "d" der die einzelnen Löcher (4) verbindenden Stege (7) grösser ist.

14. Trochanterstabilisierungsvorrichtung (40) nach einem der Ansprüche 1 bis 13,
**dadurch gekennzeichnet, dass** die Arme (4) quer zur Längsachse (8) der zentralen Platte (2) durch Stege (7) verbunden sind.

15. Trochanterstabilisierungsvorrichtung (40) nach einem der Ansprüche 1 bis 14, **dadurch gekennzeichnet, dass** die Länge der Arme (4) mindestens 6 mm, vorzugsweise mindestens 8 mm beträgt. ,

16. Trochanterstabilisierungsvorrichtung (40) nach einem der Ansprüche 1 bis 15, **dadurch gekennzeichnet, dass** jeder Arm (4) mindestens ein Loch (5) aufweist.

17. Trochanterstabilisierungsvorrichtung (40) nach einem der Ansprüche 1 -bis 16, **dadurch gekennzeichnet, dass** der Abstand zwischen zwei Löchern (5) des gleichen Arms (4) weniger als 6 mm, vorzugsweise zwischen 3,5 mm und 4,5 mm beträgt.

18. Trochanterstabilisierungsvorrichtung (40) nach einem der Ansprüche 1 bis 17, **dadurch gekennzeichnet, dass** der Abstand zwischen dem - von der Platte (2) aus gesehen - periphersten Loch (4) und der Längsachse (8) zwischen 10 mm und 40 mm beträgt.

19. Trochanterstabilisierungsvorrichtung (40) nach einem der Ansprüche 1 bis 18, **dadurch gekennzeichnet, dass** die Knochenstabilisierungsmittel (1) und die Knochenplatte (30) aufeinander abgestimmte Befestigungsperforationen (13;33) aufweisen, in welche Knochenfixationsmittel (20) einführbar sind, so dass die Stabilisierungsplatte (1) und die Knochenbefestigungslasche (30) gemeinsam mit dem Knochen (10) verbindbar sind.

20. Hüftschraubenvorrichtung mit einer Trochanterstabiliserungsvorrichtung (40) nach einem der Ansprüche 1 bis 19 und einem Befestigungselement (50),
**dadurch gekennzeichnet, dass**
das Befestigungselement (50) eine Hüftschraube oder eine Spiralklinge ist.

## Claims

1. Trochanter stabilization device (40), especially for the fixation of bone fragments in the area of the hip joint (11) or for fixing the greater trochanter (12) in position, with.
A) bone stabilization means (1), which comprise a central plate (2) with at least one fastening perforation (13) for accommodating a bone fixation means (20), and
B) a longitudinal bone plate (30), to which a sleeve (31) for accommodating a fastening element (50), which is to be introduced in the region of the hip joint (11), is fastened at an angle,
C) at least three peripheral arms (4) arise from the central plate (2), and
D) each peripheral arm (4) having at least one hole (5) for accommodating a bone fixation means (20),
**characterized in that**
E) at least a portion of the holes (5) is provided with an internal thread (6).

2. The trochanter stabilization device (40) of claim 1, **characterized in that** the central plate (2) comprises two guide rails (9), which are disposed at the longitudinal sides of the device (40) perpendicularly to the central plate (2).

3. The trochanter stabilization device (40) of claim 2, **characterized in that** the width of the bone plate (30) corresponds to the distance between the guide rails (9) in such a manner, that the central plate (2) can be pushed onto the bone plate (30) and shifted thereon in the longitudinal direction.

4. The trochanter stabilization device (40) of one of the claims 1 to 3, **characterized in that** the central plate (2) has a length L and a width B < L, as well as a longitudinal axis (8).

5. The trochanter stabilization device (40) of claim 4, **characterized in that** the arms (4) are constructed in the shape of a bone plate and have a width b < B.

6. The trochanter stabilization device (40) of one of the claims. 1 to 5, **characterized in that** the arms (4) comprise at least two sleeves (16) having the holes (5), and that the sleeves (16) can be connected with one another by means of crosspieces (7).

7. The trochanter stabilization device (40) of claim 6, **characterized in that** the sleeves (16) have an external diameter D_{A} of between 6 and 10 mm.

8. The trochanter stabilization device (40) of one of the claims 1 to 7, **characterized in that** the distance C between two holes (5) is between 10 mm and 15 mm.

9. The trochanter stabilization device (40) of one of the claims 1 to 8, **characterized in that** the angle between the two adjacent arms (4) is at least 30° and preferably at least 40°.

10. The trochanter stabilization device (40) of one of the claims 1 to 9, **characterized in that** the arms (4) enclose an angle α, which ranges from ± 5° to ± 90°, with the longitudinal axis (8).

11. The trochanter stabilization device (40) of one of the claims 1 to 10, **characterized in that** the angle α ranges from ± 10° to ± 80°.

12. The trochanter stabilization device (40) of claim 11, **characterized in that** the internal thread (6) is conical.

13. The trochanter stabilization device (40), of one of the claims 1 to 12, **characterized in that** the thickness "D" of the arms (4), measured vertically to the plate (2) in the region of the holes (5), is greater than the thickness "d" of the crosspieces (7) connecting the individual holes (4).

14. The trochanter stabilization device (40) of one of the claims 1 to 13, **characterized in that** the arms (4) are connected transversely to the longitudinal axis (8) of the central plate (2) by crosspieces (7).

15. The trochanter stabilization device (40) of one of the claims, 1 to 14, **characterized in that** the length of the arms (4) is at least 6 mm and preferably at least 8 mm.

16. The trochanter stabilization device (40) of one of the claims 1 to 15, **characterized in that** each arm (4) has at least one hole (5).

17. The trochanter stabilization device (40) of one of the claims 1 to 16, **characterized in that** the distance between two holes (5) of the same arm (4) is less than 6 mm and preferably between 3.5 mm and 4.5 mm.

18. The trochanter stabilization device (40) of one of the claims 1 to 17, **characterized in that** the distance between the most peripheral hole (4), as viewed from the plate (2), and the longitudinal axis (8) is between 10 mm and 40 mm.

19. The trochanter stabilization device (40) of one of the claims 1 to 18, **characterized in that** the bone stabilization means (1), and the bone plate (30) have fastening perforations (13; 33), which are matched to one another and into which bone fixation means (20) can be introduced, so that the stabilization plate (1) and the bone fastening link plate (30) can be connected jointly with the bone (10).

20. Hip screw device with a trochanter stabilization device (40) of one of the claims 1 to 19 and a fastening element (50), **characterized in that** the fastening element (50) is a screw or a spiral blade.

## Revendications

1. Dispositif de stabilisation du trochanter (40), en particulier pour la fixation de fragments osseux dans la région de l'articulation de la hanche (11) ou pour la fixation du grand trochanter (12), comprenant
A) des moyens de stabilisation osseuse (1) qui comprennent une plaque centrale (2) présentant au moins une perforation pour fixation (13) servant à loger un moyen de fixation osseuse (20) ; et
B) une plaque d'ostéosynthèse longitudinale (30) à laquelle est fixée une douille (31) positionnée à un certain angle, servant à loger un élément de fixation (50) à insérer dans la région de l'articulation de la hanche (11),
dans lequel
C) au moins trois bras périphériques (4) sortent de la plaque centrale (2) ; et
D) chaque bras périphérique (4) présente au moins un trou (5) servant à loger un moyen de fixation osseuse (20) ;
**caractérisé en ce que**
E) au moins une partie des trous (5) est pourvue d'un taraudage (6).

2. Dispositif de stabilisation du trochanter (40) selon la revendication 1, **caractérisé en ce que** la plaque centrale (2) comprend deux rails de guidage (9) disposés sur ses grands côtés, perpendiculairement à la plaque centrale (2).

3. Dispositif de stabilisation du trochanter (40) selon la revendication 2, **caractérisé en ce que** la largeur de la plaque d'ostéosynthèse (30) correspond à la distance entre les deux rails de guidage (9), de telle manière que l'on peut faire coulisser la plaque centrale (2) sur la plaque d'ostéosynthèse (30) et la déplacer dans sa direction longitudinale.

4. Dispositif de stabilisation du trochanter (40) selon l'une quelconque des revendications 1 à 3, **caractérisé en ce que** la plaque centrale (2) présente une longueur L et une largeur B < L ainsi qu'un axe longitudinal (8).

5. Dispositif de stabilisation du trochanter (40) selon la revendication 4, **caractérisé en ce que** les bras (4) sont réalisés en forme de plaque d'ostéosynthèse et présentent une largeur b < B.

6. Dispositif de stabilisation du trochanter (40) selon l'une quelconque des revendications 1 à 5, **caractérisé en ce que** les bras (4) comprennent au moins deux douilles (16) présentant les trous (5) et les douilles (16) peuvent être reliées entre elles au moyen d'entretoises (7).

7. Dispositif de stabilisation du trochanter (40) selon la revendication 6, **caractérisé en ce que** les douilles (16) présentent un diamètre extérieur D_{A} compris entre 6 et 10 mm.

8. Dispositif de stabilisation du trochanter (40) selon l'une quelconque des revendications 1 à 7, **caractérisé en ce que** la distance C entre deux trous (5) est comprise entre 10 mm et 15 mm.

9. Dispositif de stabilisation du trochanter (40) selon l'une quelconque des revendications 1 à 8, **caractérisé en ce que** l'angle entre les deux bras voisins (4) est d'au moins 30°, de préférence d'au moins 40°.

10. Dispositif de stabilisation du trochanter (40) selon l'une quelconque des revendications 1 à 9, **caractérisé en ce que** les bras (4) forment avec l'axe longitudinal (8) un angle α qui est dans une gamme de 5° à 90°.

11. Dispositif de stabilisation du trochanter (40) selon la revendication 10, **caractérisé en ce que** l'angle α est dans une gamme de 10° à 80°.

12. Dispositif de stabilisation du trochanter (40) selon la revendication 11, **caractérisé en ce que** le taraudage (6) est conique.

13. Dispositif de stabilisation du trochanter (40) selon l'une quelconque des revendications 1 à 12, **caractérisé en ce que** l'épaisseur "D", mesurée perpendiculairement à la plaque (2), des bras (4) dans la zone des trous (5) est plus grande que l'épaisseur "d" des entretoises (7) reliant les trous individuels (5).

14. Dispositif de stabilisation du trochanter (40) selon l'une quelconque des revendications 1 à 13, **caractérisé en ce que** les bras (4) sont reliés par des entretoises (7) transversalement à l'axe longitudinal (8) de la plaque centrale (2).

15. Dispositif de stabilisation du trochanter (40) selon l'une quelconque des revendications 1 à 14, **caractérisé en ce que** la longueur des bras (4) est d'au moins 6 mm, de préférence d'au moins 8 mm.

16. Dispositif de stabilisation du trochanter (40) selon l'une quelconque des revendications 1 à 15, **caractérisé en ce que** chaque bras (4) présente au moins un trou (5).

17. Dispositif de stabilisation du trochanter (40) selon l'une quelconque des revendications 1 à 16, **caractérisé en ce que** la distance entre deux trous (5) du même bras (4) est inférieure à 6 mm, de préférence comprise entre 3,5 mm et 4,5 mm.

18. Dispositif de stabilisation du trochanter (40) selon l'une quelconque des revendications 1 à 17, **caractérisé en ce que** la distance entre le trou (4) le plus à l'extérieur, vu depuis la plaque (2), et l'axe longitudinal (8) est comprise entre 10 mm et 40 mm.

19. Dispositif de stabilisation du trochanter (40) selon l'une quelconque des revendications 1 à 18, **caractérisé en ce que** les moyens de stabilisation osseuse (1) et la plaque d'ostéosynthèse (30) présentent des perforations de fixation (13 ; 33) adaptées les unes aux autres, dans lesquelles des moyens de fixation osseuse (20) peuvent être insérés, de sorte que la plaque de stabilisation (1) et l'éclisse de fixation osseuse (30) peuvent être conjointement reliés à l'os (10).

20. Dispositif pour vis de hanche comprenant un dispositif de stabilisation du trochanter (40) selon l'une quelconque des revendications 1 à 19 et un élément de fixation (50),
**caractérisé en ce que**
l'élément de fixation (50) est une vis de hanche ou une lame hélicoïdale.
